# EUROPEAN PATENT APPLICATION

(11) **EP 4 230 136 A1**
(43) Date of publication of application: **23.08.2023**
(21) Application number: 23156541.7
(22) Date of filing: 14.02.2023
(51) Int. Cl.: A61B 5/251, A61B 5/29, A61B 5/293, A61B 5/00

(54) **ATTACHMENT FOR A SKULL OF A RAT AND METHOD FOR INSTALLING THE ATTACHMENT**

(30) Priority: 18.02.2022 US 202217674970
(71) Applicant: Totah, Nelson, 00790 Helsinki (FI)
(72) Inventor: TOTAH, Nelson, 00790 Helsinki (FI); WATANABE, Masataka, Tokyo, 113-8654 (JP)
(74) Representative: Laine IP Oy

(57) **Abstract**

According to an example aspect of the present invention, there is provided an attachment and method for head-fixing rats for experimental purposes. The invention provides a tool for multi-region electrophysiology & precision monitoring of organismal state during complex cognitive tasks. The attachment for a skull of a rat, comprises a frame enclosing an open space inside the frame, at least one tooth on the frame extending from the frame towards the open space. Adhesive may be used as an attaching agent.

## Description

### FIELD

The invention relates to scientific studies performed on animals. More particularly, the invention relates to studies in the field of neuroscience performed on rats. Specially the invention relates to studies wherein head-fixation of the animal is required

### BACKGROUND

Head-fixation is a widely employed technique in neuroscience: it allows a combination of physiological, cellular and molecular manipulations along with behavioural observation of spontaneous or conditioned responses on a precise spatiotemporal scale. As the head is immobilized, it simplifies several recording techniques such as electrophysiology, two-photon microscopy or functional magnetic resonance imaging. While spherical treadmills are widely used in mouse models, there are only a few experimental setups suitable for adult rats, and none of them include head-fixation.

Rats are an attractive target for head-fixation, but their larger weight and power is prohibitive. There is evolutionary divergence between rat and mouse. Because of that there are physiological and functional differences, whereby these animals are more suitable for different studies, for example relating to social behaviour, drug self-administration, problem solving strategies, environmental exploration and impulsive behaviour. Methods and arrangements for head-fixation of rats has been disclosed, for example in: Journal of Neuroscience Methods; A spherical treadmill system to train head-fixed adult rats; Anabel M.M. Miguelez Fernández, Ariel Burman, Alfredo I. Martinez Cáceres, Camilo J. Mininni, B. Silvano Zanutto, Sergio E. Lew; Available online 26 December 2017, and Somatosensory and Motor Research, December 2010; 27(4): 131-148; The head-fixed behaving rat - Procedures and pitfalls; Cornelius Schwarz, Harald Hentschke, Sergejus Butovas, Florent Haiss, Maik C. Stuutgern, Todor V.Gerjikov, Caroline G. Bergner & Christian Waiblinger.

Despite the abovementioned attempts for providing head-fixation methods for rats, further development is needed to provide improved arrangements and methods for head fixation of rats.

### SUMMARY OF THE INVENTION

The invention is defined by the features of the independent claims. Some specific embodiments are defined in the dependent claims.

According to a first aspect of the present invention, there is provided an attachment for a skull of a rat, comprising:
- a frame enclosing an open space inside the frame,
- at least one tooth on the frame extending from the frame towards the open space.

According to a second aspect of the present invention, there is provided a method for attaching an attachment on a skull of a rat, comprising exposing the skull for attaching a frame on the skull, covering the skull at least partially with an (1: UV-curing joining agent, or 2: glue for attaching the frame on the skull), placing the frame on the skull and curing the (1: UV-curing joining agent, or 2: glue for attaching the frame on the skull).

According to a third aspect of the invention the attachment comprises glue for attaching the frame at least at the teeth to the skull of a rat.

According to a fourth aspect of the invention, the frame comprises multiple teeth.

According to a fifth aspect of the invention, the attachment comprises a lid for covering the open space, configured to be detachably attached to the frame.

According to a sixth aspect of the invention, the lid is attached to the frame by screws.

According to seventh aspect of the invention the frame and the teeth are one single piece of material.

According to an eight aspect of the invention, the frame and teeth are made by 3D-printing of biocompatible plastic.

### BRIEF DESCRIPTION OF THE DRAWINGS

FIGURE 1 illustrates a prior art research assembly for head-fixed mice.
FIGURE 2 illustrates in a first view a frame attached on a skull of a rat in accordance with at least some embodiments of the present invention;
FIGURE 3 illustrates in a frame attached on a skull of a rat in accordance with at least some embodiments of the present invention, shown above the skull;
FIGURE 4 illustrates the frame attached on a skull of a rat in accordance with at least some embodiments of the present invention and mounted on a halter assembly; and
FIGURE 5 illustrates an example apparatus capable of supporting at least some embodiments of the present invention.

### EMBODIMENTS

### DEFINITIONS

In the present context, the term "skull side" means a part that is configured to face a skull when mounted on the skull and term "top side" means the side opposite thereto and facing away from the skull.

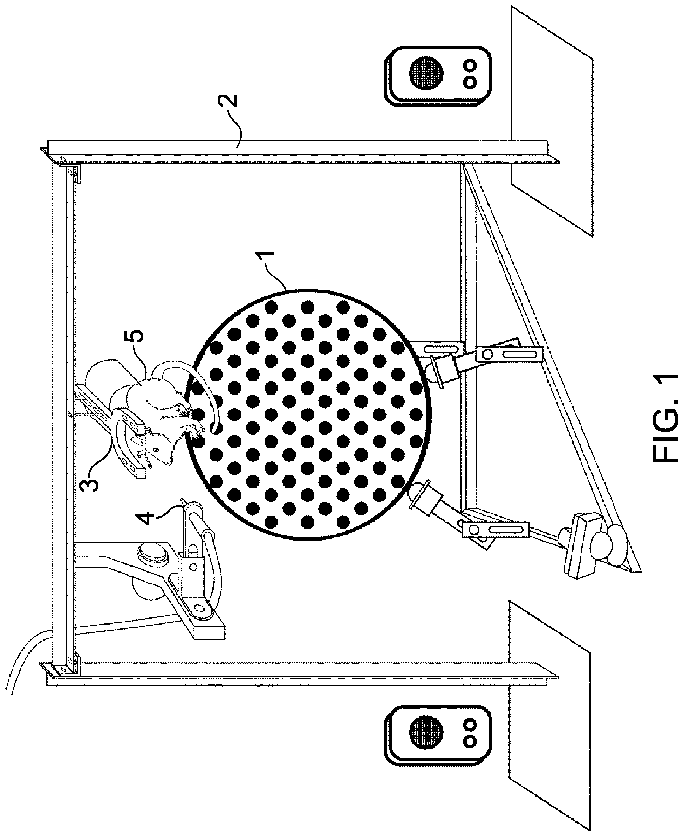

The purpose of the invention is to provide an attachment and method for head-fixing rats for experimental purposes. The invention provides a tool for multi-region electrophysiology & precision monitoring of organismal state during complex cognitive tasks. The attachment is based on a frame enclosing an open space inside the frame. The frame includes at least one tooth or usually several teeth that are integral with the frame and extend from the frame towards the open space. The open space forms a chamber-like limited area that is open in the direction away from the skull when the frame is placed over the skull of a rat. This open space or chamber provides a mounting area for instrumentation, for example, sensing and detection devices so that most of the skull is accessible for electroencephalogram (EEG) electrode array covering most of the cerebral cortex, or multiple craniometrics through which multi-electrode probes, optical fibers and drug injection cannulae, etc can be inserted for studies of physiology during behaviour studies. The teeth extend the attachment area of the frame to the skull. Large enough attachment area is needed as a rat is relatively heavy and powerful animal and a solid mount is needed to provide a head-fix that reliable and comfortable for the animal. The frame may be mounted on the skull without detection devices or the detection devices used may be mounted in the same process as the frame. Mounting the frame only allows training of the animal without detection devices while keeping the access to the skull available for attaching the detection and monitoring devices after a training period. FIGURE 1 shows a research assembly for head-fixed rats (Journal of Neuroscience Methods; A spherical treadmill system to train head-fixed adult rats; Anabel M.M. Miguelez Fernández). In this case, the assembly includes a spherical treadmill 1, a chassis 2 for an halter device 3 for holding the head of a rat 5 and monitoring and probing apparatus 4. The rat 5 is placed on the spherical treadmill 1 and moves on the surface of the treadmill 1. The head of the rat 5 is held steady by the halter device 3 to enable monitoring of its brain activity, behaviour and other responses that are experimented. This assembly is disclosed herein as a background material to illustrate the working principle of apparatus used in such experiments The head-fixes used herein is a cross-member that extends over the skull. This limits the use of the skull area and covers most of the skull and does not allow access for other implants for physiology, molecular studies, etc. A head-fix for rats that provides improved access over the skull is described below

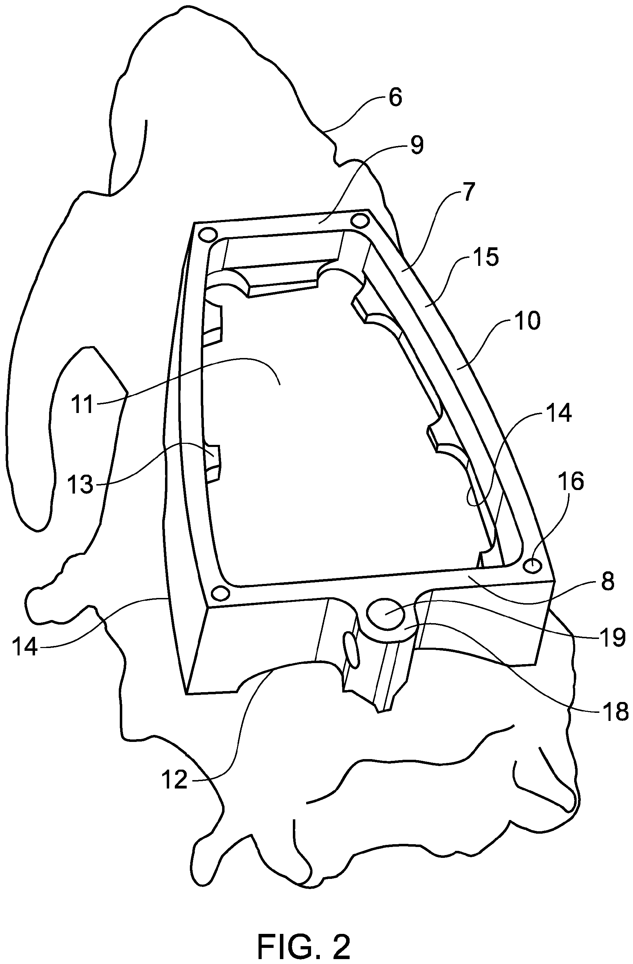

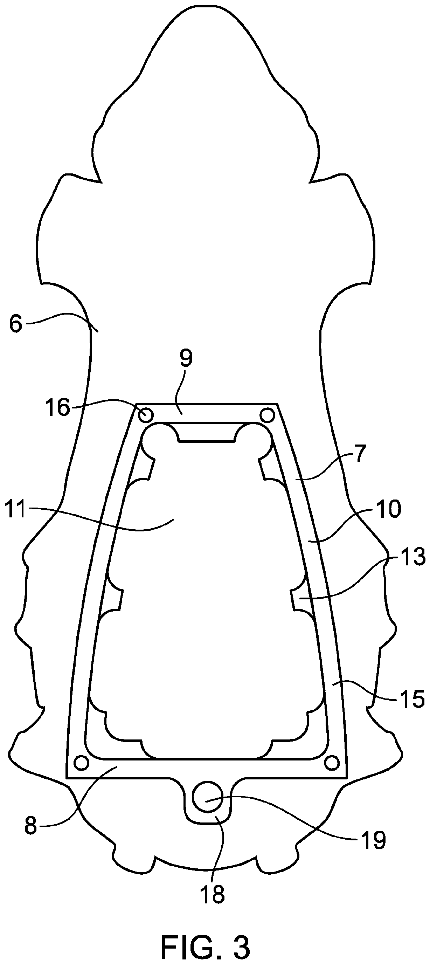

FIGURES 2 and 2 and 3 show a frame 7 on a skull 6. In this example, the frame 7 has one long straight edge 8 and a short straight edge 9 opposite to the long straight edge 8. The long straight edge 8 and the short straight edge 9 are connected with side edges 10. The side edges 10 connect to the ends of the long straight edge 8 and short straight edge 9, respectively. The edges 8, 9, 10 limit an open space 11 within them so that the frame 7 has a form of an open ring or a collar. The side edges 10 curve slightly away from the open space 11. This shape allows the frame 7 to accommodate to the outer form of the skull 6. The skull 6 is wider at the back and narrows to the front, whereby a long straight edge 8 and a narrow straight edge 9 are used. The curvature of the side edges 10 is to fit the frame 7 to the protruding top of the skull 6. This is one example of the shape of the frame 7. One can understand that the shape can be varied according to the type and shape of the instrumentation that is placed in the open space 11and the shape of the skull 6. For example, the faces of the frame 7 facing the skull when mounted, may have a curvature to accommodate the shape of the top of the skull. This is illustrated by a curved cut 12 on the skull-facing surface of the long straight edge. The short straight edge 9 and the side edges 10 may include corresponding curved cuts or other form to fit the skull side 14 of the frame to the shape of a skull.

The thickness of the edges 8, 9 and 10 of the frame 7 should be kept as small as possible. The minimum thickness is limited by structural strength requirements. In order to provide a solid structure a closed shape as described above is preferred to a shape that might have an opening slot in one or more of the edges 8, 9, 10 or a frame 7 that is formed of more than one piece. A slot, a partial cut or a hole in one of the edges 8, 9, 10 might be useful for threading wiring or fibres. However, if the thickness of the frame is small, the contact surface on the skull 6 is limited. This might compromise reliable attachment to the skull 6. In order to provide an enlarged contact surface, teeth 13 are provided on the inner wall of the frame 7. The teeth 13 are formed on the inner wall of the frame 7 at the edge on the skull side 14 of the frame 6. The teeth 13 extend towards the open space 11 formed within the frame from the inner wall of the frame 6. In this example, there is one tooth on the short straight edge 9, two teeth on each of the side edges 10 and one tooth at each corner of the long short edge 8 and straight edges 10. The shape and location of the teeth 13 is designed to accommodate the instrumentation to be used. The teeth should cover a minimum area over the skull without sacrificing reliable mounting. One design guideline that can be contemplated is to cover all or most of the area not needed for instrumentation with teeth. The tooth placement shown herein is suitable for placing an EEG array on the skull.

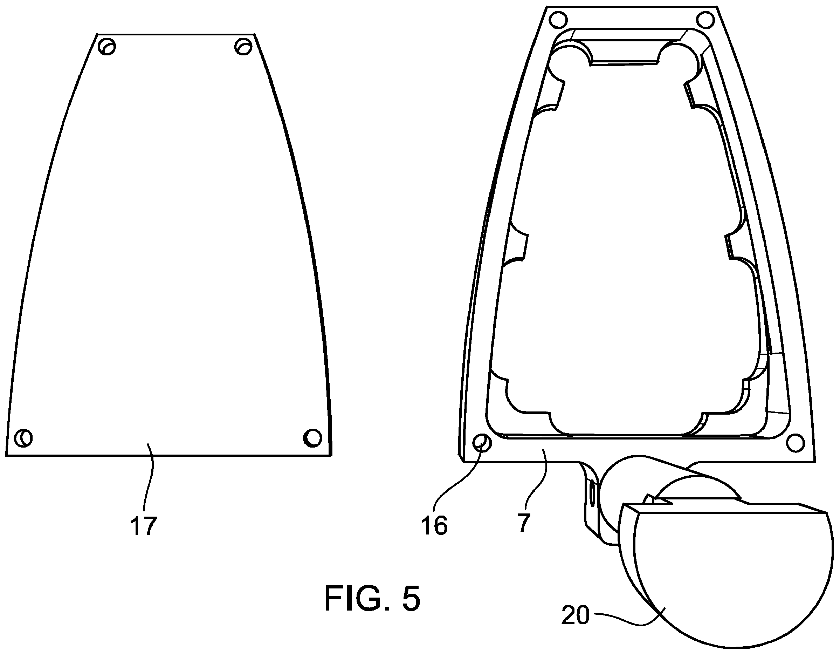

Each corner of the frame 6 has screw holes 16. These are used for attaching a lid 17 (see FIGURE 5) on the top side of the frame 6. The purpose of the lid 17 is to protect the space inside the frame 6. The function and uses of the lid 17 are described further below. For fixing the frame to a holding arrangement, a mount is needed. In this example the mount 18 is placed in the middle of the long straight edge 8. It is a ring extending from the outer surface of the long straight edge 8 and comprises a circular mounting hole 19 for a head post 20. The location of the mount at the center of the long straight edge 8 has the advantage that the mounting point is placed close to the joint between the skull and the vertebrae. This keeps the forces to the mount (and the skull) in minimum, as the support and turning point are close to each other. A mounting hole at the short straight edge 9, (at the frontal part of the skull is also applicable).

The frame 7 can be mounted on the skull 6 without instrumentation and the instrumentation added later or the mounting of instrumentation and the frame can be done at the same time. These methods are described below.

According to one method for attaching a head-fix on a skull 6 of a rat, the skull 6 is first exposed for attaching a frame 7 on the skull. The exposure is done by opening the skin on top of the skull by conventional surgical methods. Next the skull 6 is covered at least partially with, for example, an UV-curing joining agent, or other type of glue for attaching the frame on the skull. One example of the glue or joining agent is a dental cement that is cured by UV-light. These types of cements provide proven biocompatibility and controlled curing by using the UV-light. The joining agent is applied on the skull 6 or on the skull side 14 of the frame 7 or on both, depending on the type of the joining agent and its instructions of use. After application of the joining agent, the frame 7 is placed on the skull 6 and the joining agent is cured. Now the frame is solidly fixed on the skull and the open space 11 can be filled with bio-compatible silicone and the frame 7 closed with a lid 17. This procedure allows premounting of the frame 7 and training of the animal for specific tasks before mounting of the instrumentation. As the training may take several weeks, the quality of the connections of the instrumentation may deteriorate during training period. When the frame 7 is filled with silicone and closed with a lid 17, the skull is protected. The instrumentation can be installed by removing the lid 17 and silicone. Craniotomy and implantation is done after training the animal in a behavioural task. The skull under the silicone is ready for installation of the required instrumentation and as the installation is done close to the beginning of the experimentation, the risk for broken or compromised instrumentation or other malfunction is low.

The opened skin on the skull is attached to the outer sides of the frame to provide a hygienic closure. This can be done by gluing the skin to the frame 7 by a tissue glue. The gluing can be done at any time after attaching the frame.

Next, a procedure for installing the frame and the instrumentation is described. This procedure is only one illustrative example of how the invention may be implemented. The description includes several detailed steps. One or more of the steps may be omitted depending of the instrumentation to be used and sequence of he steps may be altered. Further, this example relates to implanting an EEG array. Other types of instrumentation can be used instead, but the basic principles relating to mounting of the frame are the same.

The procedure begins by providing a frame 7 described above. The surgical installation process continues as follows:
1: The skin on the top of the skull is cut open and the top of the skull is opened. The open area has to be large enough to accommodate the frame 7.
2: An EEG array I spaced on the skull and fixed with dental cement.
3: The array and the skull (top of the skull) are covered with UV-curing primer.
4: The primer is UV-cured.
5: The EEG connector is threaded through the frame and the frame is set on the skull.
6: UV-curing cement is applied to the chamber and the UV-curing cement is cured to fix the frame on the skull.
7: Installation of measuring device
8: The open space of the frame is filled with cement (for example by trade name PALADUR).
9: Skin on the skull is glued on the frame (implant) by tissue glue.
10: Cement is applied to cover exposed wires.
11: A lid may be applied.

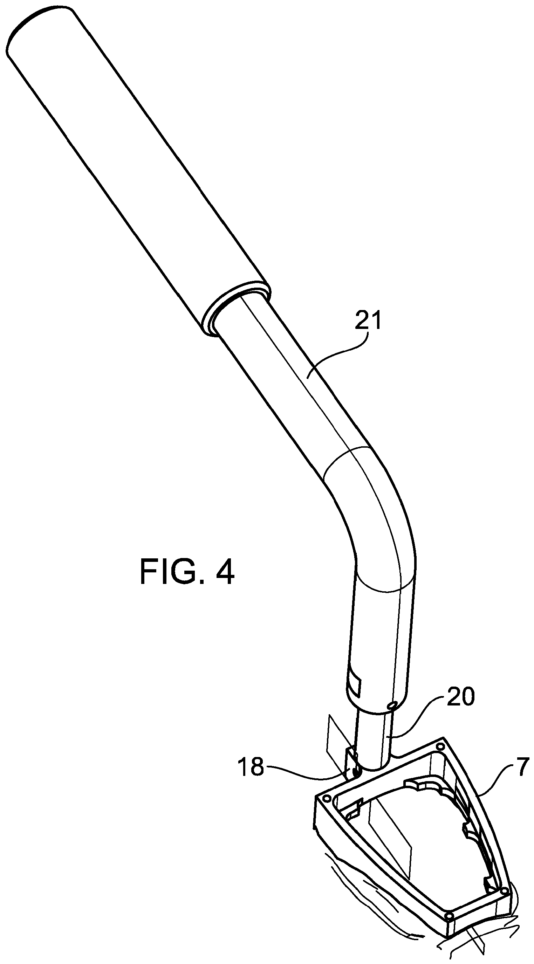

FIGURE 4 shows a halter for accomplishing the head-fixation by using the attachment described above. A head post 20 is attached to the mount 18 on the frame 7 and the head post is further connected to a halter 21. In this case the head post 20 is a straight bar extending from the mount 18. The halter 21 connects to the head post 20. The halter 21 is an angled bar turning at an angle of about 90°. It is obvious that the head post 20 and the halter 21, and accordingly the mount 18 have to be designed according to the experimentation setup used. The assembly of FIGURE 4 is disclosed herein for illustrative purposes only, except for the feature that a head post and a halter is used. Using a separate head post and halter facilitates handling of the animals.

FIGURE 5 illustrates an attachment in accordance with at least some embodiments of the present invention. Herein a frame7 as described above is shown. There is also a lid 17. The lid 17 is used for closing the open space 11 of the frame 7 and has holes for screws at each corner. The screws are preferred as fixing elements herein. One can contemplate that a flexible tongue/groove locking setup or similar is used as an alternative, but operating the lid 17 when the frame and the lid are fixed on an animal may be complicated in such setups.

The frame and the cover may be manufactured by any common manufacturing methods, such a injection moulding. However, a very large scale manufacturing may not be needed whereby a 3D-printing might be a lucrative manufacturing alternative. The materials used, at least in frame and joining agents must be biocompatible. For example, anything biocompatible and lightweight that can be 3D printed or CNC milled such as PVC, titanium, etc. can be used. Dental cements and corresponding adhesives can be used for fixing the frame to the skull.

A head-fixation attachment described herein enables `whole brain' access and the head-fixation is low stress. During experimentation, 7% failure rate (implants lost) for daily experiments up to 3.5 months for 126 trained rats was discovered, up to 8 months has been tested. The attachment and method provides improvement of SNR in comparison to freely moving rats. Tools to monitor arousal, organism state, and instrumental responses and to present full-field visual stimulation can be provided using the attachment.

Ideal used of the attachment and the method are considered to be:
- High signal to noise ratio neurophysiology
- Complex cognitive tasks
- Control animals's physical orientation
- Precise stimulus onset timing
- Less variable behavioural responses
- Measurement of organism state (oro-facial movements, locomotion, grooming, yawning, etc.)

It is to be understood that the embodiments of the invention disclosed are not limited to the particular structures, process steps, or materials disclosed herein, but are extended to equivalents thereof as would be recognized by those ordinarily skilled in the relevant arts. It should also be understood that terminology employed herein is used for the purpose of describing particular embodiments only and is not intended to be limiting.

Reference throughout this specification to "one embodiment" or "an embodiment" means that a particular feature, structure, or characteristic described in connection with the embodiment is included in at least one embodiment of the present invention. Thus, appearances of the phrases "in one embodiment" or "in an embodiment" in various places throughout this specification are not necessarily all referring to the same embodiment.

As used herein, a plurality of items, structural elements, compositional elements, and/or materials may be presented in a common list for convenience. However, these lists should be construed as though each member of the list is individually identified as a separate and unique member. Thus, no individual member of such list should be construed as a de facto equivalent of any other member of the same list solely based on their presentation in a common group without indications to the contrary. In addition, various embodiments and example of the present invention may be referred to herein along with alternatives for the various components thereof. It is understood that such embodiments, examples, and alternatives are not to be construed as de facto equivalents of one another, but are to be considered as separate and autonomous representations of the present invention.

Furthermore, the described features, structures, or characteristics may be combined in any suitable manner in one or more embodiments. In the following description, numerous specific details are provided, such as examples of lengths, widths, shapes, etc., to provide a thorough understanding of embodiments of the invention. One skilled in the relevant art will recognize, however, that the invention can be practiced without one or more of the specific details, or with other methods, components, materials, etc. In other instances, well-known structures, materials, or operations are not shown or described in detail to avoid obscuring aspects of the invention.

While the forgoing examples are illustrative of the principles of the present invention in one or more particular applications, it will be apparent to those of ordinary skill in the art that numerous modifications in form, usage and details of implementation can be made without the exercise of inventive faculty, and without departing from the principles and concepts of the invention. Accordingly, it is not intended that the invention be limited, except as by the claims set forth below.

The verbs "to comprise" and "to include" are used in this document as open limitations that neither exclude nor require the existence of also un-recited features. The features recited in depending claims are mutually freely combinable unless otherwise explicitly stated. Furthermore, it is to be understood that the use of "a" or "an", i.e. a singular form, throughout this document does not exclude a plurality.

### INDUSTRIAL APPLICABILITY

The invention can be used in providing apparatuses for experimental setups.

### ACRONYMS LIST

- EEG: Electroencephalogram
- GND: Ground
- EIB: Electrode Interface Board
- SNR: Signal Noise Ratio

### REFERENCE SIGNS LIST

- 1: spherical treadmill
- 2: chassis
- 3: halter device
- 4: probing apparatus
- 5: mouse
- 6: skull
- 7: frame
- 8: long straight edge
- 9: short straight edge
- 10: side edge
- 11: open space
- 12: curved cut
- 13: teeth
- 14: skull side
- 15: top side
- 16: screw hole
- 17: lid
- 18: mount
- 19: mounting hole
- 20: head post
- 21: halter

### CITATION LIST

### Non Patent Literature:

McGinlay, MJ, et al. 2015, Neuron 87, 179-192
Journal of Neuroscience Methods; A spherical treadmill system to train head-fixed adult rats; Anabel M.M. Miguelez Fernández, Ariel Burman, Alfredo I. Martinez
Cáceres,Camilo J. Mininni, B. Silvano Zanutto, Sergio E. Lew; Journal homepage: www.elsevier.com/locate/jneumeth; Available online 26 December 2017
Somatosensory and Motor Research, December 2010; 27(4): 131-148; The head-fixed behaving rat - Procedures and pitfalls; Cornelius Schwarz, Harald Hentschke, Sergejus Butovas, Florent Haiss, Maik C. Stuutgern, Todor V.Gerjikov, Caroline G. Bergner & Christian Waiblinger

## Claims

1. An attachment for a skull of a rat, comprising:
- a frame enclosing an open space inside the frame, and
- at least one tooth on the frame extending from the frame towards the open space.

2. The attachment according to claim 1, further comprising a joining agent for attaching the frame at least at the at least one tooth to the skull of the rat.

3. The attachment according to any one of claims 1-2, wherein the frame comprises multiple teeth.

4. The attachment according to any one of claims 1-3, wherein the attachment comprises a lid for covering the open space, configured to be detachably attached to the frame.

5. The attachment according to claim 4, wherein the lid is attached to the frame by screws.

6. The attachment according to any one of claims 1-5, wherein the frame and the at least one tooth are one single piece of material.

7. The attachment according to any one of claims 1-6, wherein the frame and the at least one tooth are 3D-printed from a biocompatible plastic.

8. The attachment according to claim 2, wherein the joining agent is UV-curable cement, for example, a dental cement.

9. A method for attaching a head-fix on a skull of a rat, comprising:
- exposing the skull,
- covering the skull at least partially with a joining agent,
- placing a frame on the skull, wherein the frame encloses an open space inside the frame, and comprises at least one tooth on the frame extending from the frame towards the open space, and
- curing the joining agent to attach the frame on the skull.

10. The method according to claim 9, further comprising performing craniotomy and implantation, for example, for attaching an EEG array on the skull before attachment of the frame.

11. The method according to claim 9, comprising:
- filling the frame with biocompatible silicone after attaching the frame on the skull,
- covering the open space within the frame by a lid,
- removing the lid, and
- performing craniotomy and implantation, for example, for attaching an EEG array on the skull.
